# EUROPEAN PATENT SPECIFICATION

(11) **EP 2 398 521 B1**
(45) Date of publication and mention of the grant of the patent: **01.11.2017**
(21) Application number: 10705056.9
(22) Date of filing: 18.02.2010
(51) Int. Cl.: A61L 31/02

(54) **BIOERODIBLE ENDOPROSTHESIS**
BIOERODIERBARE ENDOPROTHESE
ENDOPROTHÈSE BIOÉRODABLE

(30) Priority: 20.02.2009 US 389792
(43) Date of publication of application: 28.12.2011
(73) Proprietor: Boston Scientific Scimed, Inc., Maple Grove, MN 55311-1566 (US)
(72) Inventor: SCHEUERMANN, Torsten, 80803 Munich (DE); WEBER, Jan, 6228 GJ Maastricht (NL); ALBRECHT, Peter, 82340 Feldafing (DE)
(74) Representative: Peterreins Schley
(86) International application number: PCT/US2010/024520
(87) International publication number: WO 2010/096516

(56) References cited:
- US-A1- 2007 250 155
- US-A1- 2009 030 500

## Description

### TECHNICAL FIELD

The present invention relates to endoprostheses, and more particularly to stents.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body. Examples of endoprostheses include stents, covered stents, and stent-grafts.

Endoprostheses can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

The expansion mechanism can include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn.

In another delivery technique, the endoprosthesis is formed of an elastic material that can be reversibly compacted and expanded, e.g., elastically or through a material phase transition. During introduction into the body, the endoprosthesis is restrained in a compacted condition. Upon reaching the desired implantation site, the restraint is removed, for example, by retracting a restraining device such as an outer sheath, enabling the endoprosthesis to self-expand by its own internal elastic restoring force.

It is sometimes desirable for an implanted endoprosthesis to erode over time within the passageway. For example, a fully erodable endoprosthesis does not remain as a permanent object in the body, which may help the passageway recover to its natural condition. Erodible endoprostheses can be formed from, e.g., a polymeric material, such as polylactic acid, or from a metallic material such as magnesium, iron or an alloy thereof.

US 2009/030500 A1 discloses an endoprosthesis that includes a base portion and a source of Fe(II) ions that is compositionally distinct from the base portion and releasable from the endoprosthesis under physiological conditions.

US 2007/250155 A1 discloses an implantable medical device is provided that degrades upon contact with body fluids so as to limit its residence time within the body. The device is formed of an iron carbon alloy that is subjected to DET heat treatment to impart high strength and high ductility in combination with an accelerated corrosion rate.

### SUMMARY

The present invention is directed to an endoprosthesis, more specifically, a biodegradable stent.

In a first aspect, the invention features a medical stent as further defined in appended claim 1.

In another aspect, the invention features an apparatus ias further defined in appended claim 3.

In another aspect, the invention features a method of forming a as further defined in appended claim 4.

Embodiments may also include one or more of the following features. The composition includes 90 % or more Fe, 0.5-6% Mn, 0.001%-3% Si, and 0.1 % or less C. The composition has a degradation rate of about 60 micron (µm) per year or greater. The composition has a degradation rate of about 130 micron (µm) per year or greater. The composing has a degradation rate greater than iron by about 10% or more. The composition has a yield strength of about 250-450 MPa, an elongation to break of about 15% or greater, an area reduction of about 50% or less, and a ductility of about 30% or more. The composition can consist essentially of or consist of any of the element combinations described herein.

Embodiments may additionally include one or more of the following features. The stent body has a wall thickness of about 150 micron (µm) or less. The body of the stent has a delivery diameter of about 1mm to about 5mm. The body of the stent has a delivery diameter of about 5 mm or greater.

Aspects, embodiments or implementations may include one or more of the following advantages. A stent includes a metal composition that has advantageous mechanical properties for reducing the likelihood of restenosis, a low profile, and a desirable degradation rate. In particular embodiments, the alloy composition allows for a bioerodible stent with mechanical and dimensional properties similar to stainless steel stents. The Iron -alloy composition also allows for a stent having similar strength to stents of pure iron, but with a significant reduction in total volume of the stent. A smaller volume reduces the amount of corrosion products in the patient. The composition can degrade faster than iron, e.g., about 5-20% faster. The composition has a high yield strength and is biocompatible. The stents can be made by known processing techniques such as drawing, laser cutting, and electropolishing. The composition has high ductility, allowing stent designs usually intended for stainless steel and other biostable alloys, including relatively thin, narrow struts and high expansion ratios.

The endoprosthesis may not need to be removed from a lumen after implantation. The endoprosthesis can have a low thrombogenecity and high initial strength. The endoprosthesis can exhibit reduced spring back (recoil) after expansion. Lumens implanted with the endoprosthesis can exhibit reduced restenosis. The endoprosthesis can be erodible. The rate of erosion of different portions of the endoprosthesis can be controlled, allowing the endoprosthesis to erode in a predetermined manner and reducing, e.g., the likelihood of uncontrolled fragmentation and embolization. For example, the predetermined manner of erosion can be from a first end of the endoprosthesis to a second end of the endoprosthesis. The controlled rate of erosion and the predetermined manner of erosion can extend the time the endoprosthesis takes to erode to a particular degree of erosion, can extend the time that the endoprosthesis can maintain patency of the passageway in which the endoprosthesis is implanted, can allow better control over the size of the released particles during erosion, and/or can allow the cells of the implantation passageway to better endothelialize around the endoprosthesis.

The details of one or more embodiments are set forth in the accompanying drawings and the description below. Other features, objects, and advantages will be apparent from the description and drawings, and from the claims.

### DESCRIPTION OF DRAWINGS

FIGS. 1A-1C are sequential, longitudinal cross-sectional views, illustrating delivery of an endoprosthesis in a collapsed state, expansion of the endoprosthesis, and the deployment of the endoprosthesis in a body lumen.
FIG. 2 is a perspective view of an embodiment of a stent.
FIGS. 3A-B is a schematic drawing illustrating a stent corrosion in a portion of the stent.
FIG. 4 is a graph of tensile results.
FIG. 4A is a schematic of a testing sample.
FIG. 5 is a graph of erosion rates.
FIG. 6 is a graph of cell inhibition tests.
FIG. 7 is a scanning electron micrograph of a stent.

### DETAILED DESCRIPTION

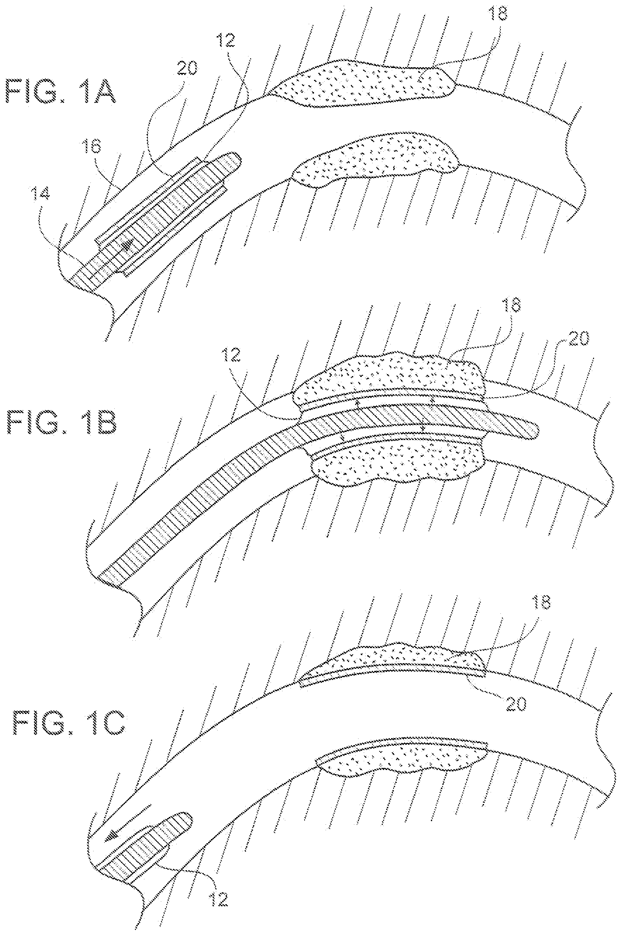

Referring to FIGS. 1A-1C, a stent 20 is placed over a balloon 12 carried near a distal end of a catheter 14, and is directed through the lumen 16 (FIG. 1A) until the portion carrying the balloon and stent reaches the region of an occlusion 18. The stent 20 is then radially expanded, e.g. by inflating the balloon 12, and compressed against the vessel wall with the result that occlusion 18 is compressed, and the vessel wall surrounding it undergoes a radial expansion (FIG. 1B). The pressure is then released from the balloon and the catheter is withdrawn from the vessel (FIG. 1C).

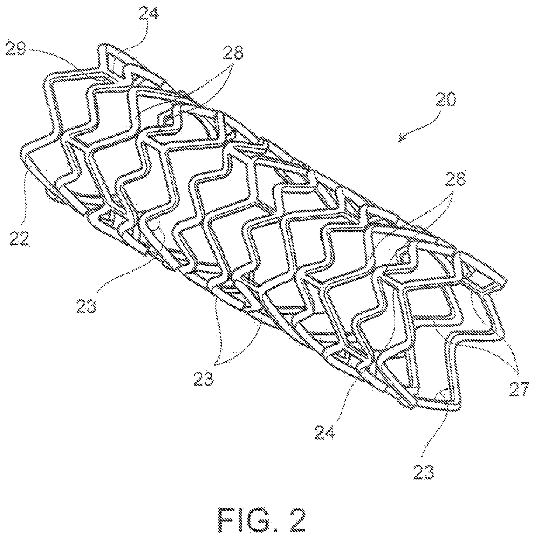

Referring to FIG. 2, an expandable stent 20 can have a stent body having the form of a tubular member defined by a plurality of bands 22 and a plurality of connectors 24 that extend between and connect adjacent bands. During use, bands 22 can be expanded from an initial, smaller diameter to a larger diameter to contact stent 20 against a wall of a vessel, thereby maintaining the patency of the vessel. Connectors 24 can provide stent 20 with flexibility and conformability that allow the stent to adapt to the contours of the vessel. One or more bands 22 form acute angles 23. The angle 23 increases upon expansion of the stent. Stent body 20, bands 22 and connectors 24 can have a luminal surface 26, an abluminal surface 28, and a sidewall surface 29. In embodiments, the bands and/or connectors, have a width across the abluminal surface, and a thickness between the abluminal and luminal surfaces, of about 50 to 150 microns.

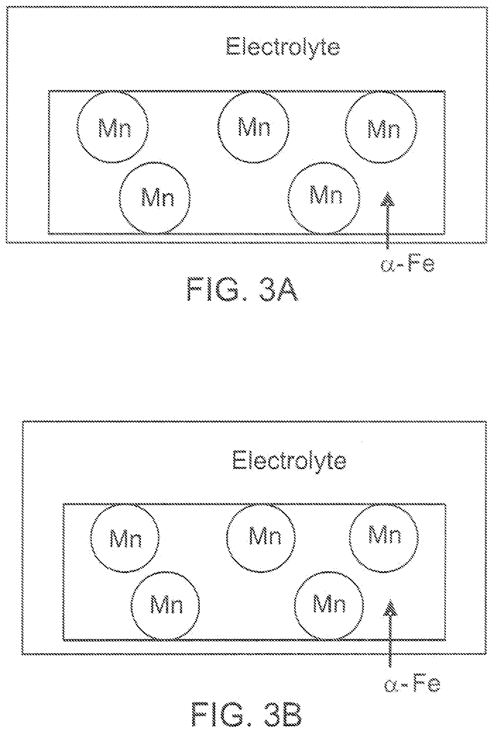

Referring to Figures 3A and 3B, the stent body 20 is formed of a metal composition of Fe and Mn. The alloying of Fe with Mn in select amount controls the erosion rate. The Mn is a less noble metal and serves as an anode in combination with Fe in the presence of an electrolyte. The anodic material is eroded initally (Fig 3A), which creates pores on the surface, (Fig. 3B). The porous surface then accelerates the corrosion rate of the Fe.

The composition can include small amounts of Si and/or C to increase strength, in particular Si is 0.001 to 3%, preferably about 0.1 to 0.3%, and C is less than 0.1%, preferably 0.01 to 0.03%. The composition may or may not include minor amounts of other elements. Examples of bioerodible alloys (including some examples not according to the invention) include iron alloys having, by weight, 90-99.5% iron, 0.5-10% manganese, 0 % -3% silicon, and 0% - 1% carbon and/or less than 5% of other elements (e.g., Silver and Platinum). Suitable alloys are included in the following table:

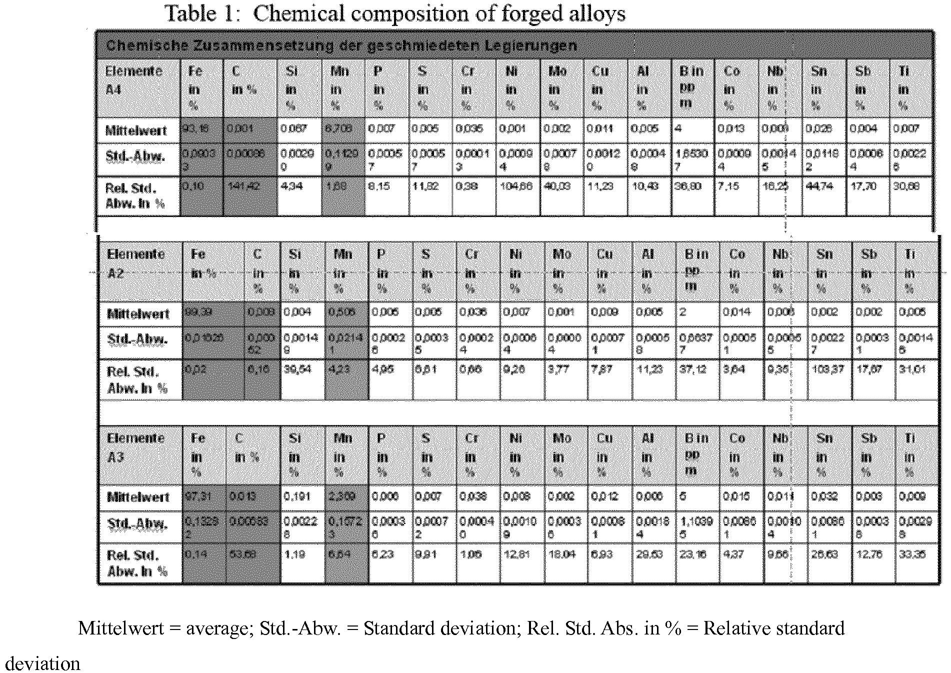

Alloys of the compositions can be purchased from Wieland Dental + Technik GmbH & Co.. KG, Schwenninger Strasse 13, D - 75179 Pforzheim, Germany. The compositions can consist essentially of or consist entirely of the element combinations described herein.

In embodiments, the composition has mechanical and degradation properties advantageous to stent treatment. The composition has a high yield strength, for example about 250 MPa or more, e.g. about 280 to 400MPa, and an elongation at break of about 12% or more, e.g. 15% or more and an area reduction of greater than about 90%. The compositions can be formed by alloying. The alloys can be drawn into tubes, laser cut and electropolished.

The composition has an average mass loss of about 650-725 µm/a when immersed in a test solution containing 0.9%NaCl as described in the Example. The corrosion rate in vivo may be significantly lower than when measured in vitro due to the formation of a biofilm (fibrinogen, albumin and extra-cellular matrix) on the surface of the implant. This reduction in rate could be more then ten fold. A stent comprising the composition can have an in vivo corrosion rate of about 50 micron (µm) per year or more, preferable 130 µm per year or more. In embodiments, the composition and stent dimensions are selected such that the stent is eroded for more than 95% of the original volume within 10 to 24 months from implantation. The stent can have a low profile, e.g. with a wall thickness of about 150 µm or less, e.g. about 80 µm or less. The alloy can be used with common stent patterns, such as the Libertè® stent pattern from Boston Scientific, Inc. For example, the struts can have a width of about 200 µm or less, e.g. about 150 µm or 100 µm or less.

In addition, the composition has an anti-proliferative effect on smooth muscle cells and endothelial cells. For example, endothelial cell ("EC") and smooth muscle cell ("SMC") cultures containing composition of binary Fe-2.7 Mn alloy have an inhibition zone surface area of about 40-64 µm² after 144 hours.

### Example:

A bioerodible alloy having the alloy composition A3 from Table I is provided: 97.31% Fe, 0.013% C, 0.191% Si, 2.369% Mn, and trace amounts of: P, S, Cr, Ni, Mo, Cu, Al, B, Co, Nb, Sn, Sb, and Ti.

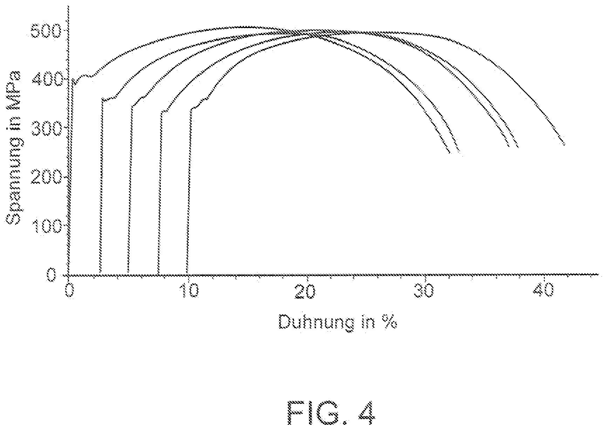

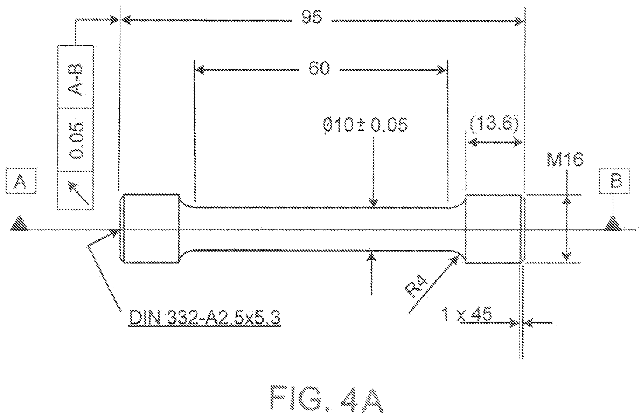

Referring to Fig. 4, the composition has a tensile strength between 400 MPa and 480 MPa at 10-30% elongation. The tests are done according to EN 10002-1 using sample geometry as described in DIN 50125-B (10 x 50) and depicted in Figure 4A. The composition also has an elongation to break of 39 %, and a reduction of area of 89 %, (the latter is measured by measuring the cross-dimensional surface area at the breaking site).

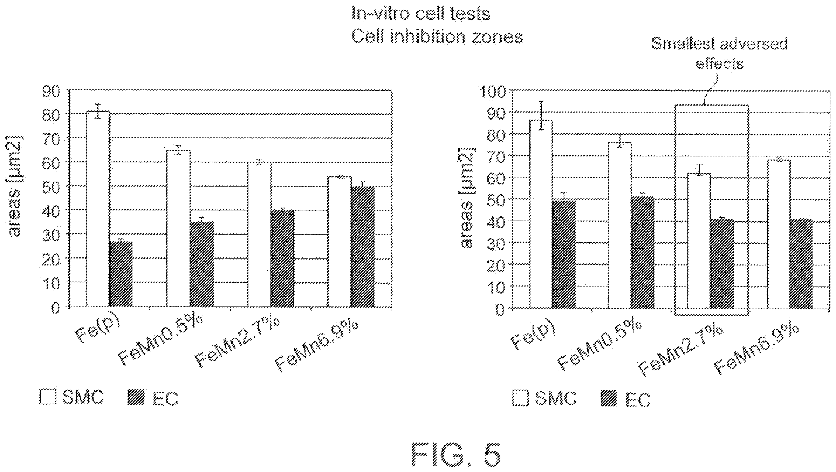

Referring to Fig. 5, the erosion rates or mass loss rates per year of various compositions are compared, wherein: Fe has a mass loss of about 620 µm/a; Fe with 0.5% Mn has a mass loss of about 660 µm/a; Fe with 2.7% Mn has a mass loss of about 705 µm/a; and Fe with 6.9% Mn has a mass loss of about 675 µm/a. To measure the erosion rate of the alloy compositions, round pellets (10 mm diameter) and rods of diameter 2 and 4 mm were made from the composition and immersed in NaCl 0.9%/pH 7+/- 0.5. The weight of the various samples were determined at one month time intervals and back calculated to an equivalent uniform surface erosion depth in microns on a year basis. Mass loss calculation is made after the cleaning of corrosion products with following method: specimen cleaned for 5 min by etching with a 3.5g Hexamethylentetramin in 500 ml 37% HCl to 11 dest. aq. solution.

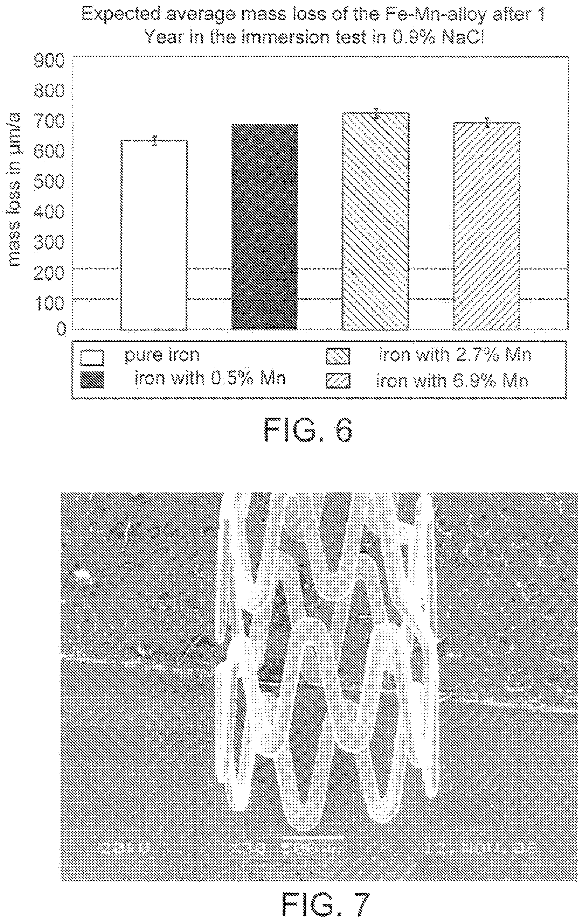

Referring to Fig. 6, human endothelial cell ("EC") and smooth muscle cell ("SMC") cultures containing pellets of the composition have an inhibition zone surface area of about 40-64 µm² after 144 hours. Inhibition zone surface area is determined by making 10 mm round pellets of the compositions and fixing the pellets using paraffin in the center of cell culture plate cavities. After seeding with endothelial cells ("EC") or smooth muscle cells ("SMC") and leaving the cell cultures for a predetermined timeframe, the perimeter surrounding these round pellets is determined. Within the perimeter there are essentially no living cells. Outside of this border or perimeter, cells show normal cell growth behavior. The average radial distance between the pellet diameter and the life\dead perimeter is measured and the annular surface area is defined as the inhibition zone surface area.

Referring to Fig. 7, a stent is shown in the unexpanded form using a scanning electron micrograph at x30 magnification. The composition is drawn into tubular form, cut into a known stent geometry, (such as the Liberte' Stent from Boston Scientific, Inc.) and electropolished, to form stent 21. Stent 21 has a recoil of about 2%, foreshortening upon expansion of about 5.7%, with a compression force of 0.28 N/mm and no fractures upon overexpansion. To test the stent's mechanical properties, the stents are crimped on to a standard balloon catheter (e.g., the Libertè®, 3.5 mm balloon catheter from Boston Scientific, Inc.) and expanded to a nominal diameter of 3.5 mm, using a nominal internal pressure in the balloon of 14 atm. The outer stent diameter is measured using a laser measurement system before and after deflating the balloon. The percentage recoil is determined by these two measures. Similarly the length of the stent is measured before and after deployment of the balloon system. The compression force is determined by placing the expanded stent in a double V-grooved assembly on a pull-bench and measuring the stress-strain curve while narrowing the distance between the upper and lower jaw.

### Other Embodiments:

A stent is bioerodible if the stent or a portion thereof exhibits substantial mass or density reduction or chemical transformation, after it is introduced into a patient, e.g., a human patient. Mass reduction can occur by, e.g., dissolution of the material that forms the stent and/or fragmenting of the stent. Chemical transformation can include oxidation/reduction, hydrolysis, substitution, and/or addition reactions, or other chemical reactions of the material from which the stent or a portion thereof is made. The erosion can be the result of a chemical and/or biological interaction of the stent with the body environment, e.g., the body itself or body fluids, into which it is implanted. The erosion can also be triggered by applying a triggering influence, such as a chemical reactant or energy to the stent, e.g., to increase a reaction rate. For example, a stent or a portion thereof can be formed from an active metal, e.g., Mg or Fe or an alloy thereof, and which can erode by reaction with water, producing the corresponding metal oxide and hydrogen gas; a stent or a portion thereof can also be formed from a bioerodible polymer, or a blend of bioerodible polymers which can erode by hydrolysis with water. Fragmentation of a stent occurs as, e.g., some regions of the stent erode more rapidly than other regions. The faster eroding regions become weakened by more quickly eroding through the body of the endoprosthesis and fragment from the slower eroding regions.

Preferably, the erosion occurs to a desirable extent in a time frame that can provide a therapeutic benefit. For example, the stent may exhibit substantial mass reduction after a period of time when a function of the stent, such as support of the lumen wall or drug delivery, is no longer needed or desirable. In certain applications, stents exhibit a mass reduction of about 10 percent or more, e.g. about 50 percent or more, after a period of implantation of about one day or more, about 60 days or more, about 180 days or more, about 600 days or more, or about 1000 days or less. Erosion rates can be adjusted to allow a stent to erode in a desired sequence by either reducing or increasing erosion rates. For example, regions can be treated to increase erosion rates by enhancing their chemical reactivity, e.g., coating portions of the stent with a silver coating to create a galvanic couple with the exposed, uncoated Iron surfaces on other parts of the stent. Alternatively, regions can be treated to reduce erosion rates, e.g., by using coatings.

A coating can be deposited or applied over the surface of stent to provide a desired function. Examples of such coatings include a tie layer, a biocompatible outer coating, a radiopaque metal or alloy, and/or a drug-eluting layer.

A stent can be incorporated with at least one releasable therapeutic agent, drug, or pharmaceutically active compound to inhibit restenosis, such as paclitaxel, or to treat and/or inhibit pain, encrustation of the stent or sclerosing or necrosing of a treated lumen. The therapeutic agent can be a genetic therapeutic agent, a non-genetic therapeutic agent, or cells. The therapeutic agent can also be nonionic, or anionic and/or cationic in nature. Examples of suitable therapeutic agents, drugs, or pharmaceutically active compounds include anti-thrombogenic agents, antioxidants, anti-inflammatory agents, anesthetic agents, anti-coagulants, and antibiotics, as described in U.S. Patent No. 5,674,242; U.S.S.N. 09/895,415, filed July 2, 2001; U.S.S.N. 11/111,509, filed April 21, 2005; and U.S.S.N. 10/232,265, filed August 30, 2002,. Representative conventional approaches disperse the therapeutic agent, drug, or a pharmaceutically active compound in a polymeric coating carried by a stent. In the present invention, the therapeutic agent, drug, or a pharmaceutically active compound can be directly incorporated into the pores generated by plasma immersion ion implantation treatment on the surface of a stent, thereby eliminating the use of extra coatings.

The materials described above can be used for the entire stent body, or a portion of the stent body, or as a layer on a stent made of another material or can include a layer of another material, which other material may be other bioerodible or biostable, a metal, a polymer or a ceramic. The stent can include in addition to the materials described above, iron or an alloy thereof. In some embodiments, the stent can include one or more bioerodible metals, such as magnesium, zinc, iron, or alloys thereof. The stent can include bioerodible and non-bioerodible materials. The stent can have a surface including bioerodible metals, polymeric materials, or ceramics. The stent can have a surface including an oxide of a bioerodible metal. Examples of bioerodible alloys also include magnesium alloys having, by weight, 50-98% magnesium, 0-40% lithium, 0-1% iron and less than 5% other metals or rare earths; or 79-97% magnesium, 2-5% aluminum, 0-12% lithium and 1-4% rare earths (such as cerium, lanthanum, neodymium and/or praseodymium); or 85-91% magnesium, 6-12% lithium, 2% aluminum and 1% rare earths; or 86-97% magnesium, 0-8% lithium, 2-4% aluminum and 1-2% rare earths; or 8.5-9.5% aluminum, 0.15%-0.4% manganese, 0.45-0.9% zinc and the remainder magnesium; or 4.5-5.3% aluminum, 0.28%-0.5% manganese and the remainder magnesium; or 55-65% magnesium, 30-40% lithium and 0-5% other metals and/or rare earths. Bioerodible magnesium alloys are also available under the names AZ91D, AM50A, andAE42. Other bioerodible alloys are described in Bolz, U.S. 6,287,332 (e.g., zinc-titanium alloy and sodium-magnesium alloys); Heublein, U.S. Patent Application 2002000406; and Park, Science and Technology of Advanced Materials, 2, 73-78 (2001). In particular, Park describes Mg-X-Ca alloys, e.g., Mg-Al-Si-Ca, Mg-Zn-Ca alloys. Examples of bioerodible polymers include polydioxanone, polycaprolactone, polygluconate, polylactic acid-polyethylene oxide copolymers, modified cellulose, collagen, poly(hydroxybutyrate), polyanhydride, polyphosphoester, poly(amino acids), poly-L-lactide, poly-D-lactide, polyglycolide, poly(alpha-hydroxy acid), and combinations thereof.

A stent can also include non-bioerodible materials. Examples of suitable non-bioerodible materials include stainless steels, platinum enhanced stainless steels, cobalt-chromium alloys, nickel-titanium alloys, noble metals and combinations thereof. In some embodiments, stent 20 can include bioerodible and non-bioerodible portions. In some embodiments, non-bioerodible or biostable metals can be used to enhance the X-ray visibility of bioerodible stents. The bioerodible stent main structure of a stent can be combined with one or more biostable marker sections. The biostable marker sections can include, for example, Gold, Platinum or other high atomic weight elements. The biostable marker sections can provide enhance visibility and radiopacity and can provide a structural purpose as well.

A stent can have any desired shape and size (e.g., superficial femoral artery stents, coronary stents, aortic stents, peripheral vascular stents, gastrointestinal stents, urology stents, and neurology stents). Depending on the application, stent 20 can have an expanded diameter of about 1 mm to about 46 mm. For example, a coronary stent can have an expanded diameter of about 2 mm to about 6 mm; a peripheral stent can have an expanded diameter of about 5 mm to about 24 mm; a gastrointestinal and/or urology stent can have an expanded diameter of about 6 mm to about 30 mm; a neurology stent can have an expanded diameter of about 1 mm to about 12 mm; and an abdominal aortic aneurysm stent and a thoracic aortic aneurysm stent can have an expanded diameter of about 20 mm to about 46 mm. Stent 20 can be self-expandable, balloon-expandable, or a combination of self-expandable and balloon-expandable (e.g., as described in U.S. Patent No. 5,366,504). Stent 20 can have any suitable transverse cross-section, including circular and non-circular (e.g., polygonal such as square, hexagonal or octagonal).

One class of stents that may benefit from the erodible nature of the stent material, would be stents intended to be used in bifurcations. The complex cyclic movement of the vessels at those spots causes a high restenosis rate when restricted in movement due to a permanent stent implant. An erodible stent has therefore a significant advantage over permanent implants. The geometry of bifurcation stents can have special sections adapted to support the ostium of the side branch. U.S. patent application Ser. No. 09/963,114, filed on Sep. 24, 2001, U.S. patent application Ser. No. 10/644,550, filed on Aug. 21, 2003, U.S. patent application Ser. No. 10/910,598, filed Aug. 4, 2004, and U.S. Patent Application Publication 20070233270 filed May 30, 2007, describe bifurcated stents . Fe-Mn-Si-C alloys having mechanical properties similar to stainless steel are suitable for use with these stent patterns.

A stent can be implemented using a catheter delivery system. Catheter systems are described in, for example, Wang U.S. 5,195,969; Hamlin U.S. 5,270,086; and Raeder-Devens, U.S. 6,726,712. Commercial examples of stents and stent delivery systems include Radius®, Symbiot® or Sentinol® system, available from Boston Scientific Scimed, Maple Grove, MN.

A stent can be a part of a covered stent or a stent-graft. For example, a stent can include and/or be attached to a biocompatible, non-porous or semi-porous polymer matrix made of polytetrafluoroethylene (PTFE), expanded PTFE, polyethylene, urethane, or polypropylene. In addition to vascular lumens, a stent can be configured for non-vascular lumens. For example, it can be configured for use in the esophagus or the prostate. Other lumens include biliary lumens, hepatic lumens, pancreatic lumens, uretheral lumens and ureteral lumens.

Still other embodiments are in the following claims.

## Claims

1. A medical stent, comprising: a tubular body formed of a metal composition consisting of Fe, Mn, Si, and C, the composition may or may not include minor amounts of other elements, wherein Fe is present in amounts of at least 90%, wherein Mn is present in amounts of 0.5-6%, Si is present in amounts of 0.001-3%, C is present in amounts of 0.01 to 0.1%.

2. The medical stent of claim 1, wherein the amounts of Mn, Si, and C in the composition are as follows: 2-3% Mn, 0.1-0.3% Si, and 0.01 - 0.03% C.

3. An apparatus comprising: a catheter and a stent according to claim 1 that is mounted on the catheter, the catheter arranged to expand the stent by plastic deformation.

4. A method for forming the stent of claim 1, comprising:
providing a metal composition consisting of Fe, Mn, Si, and C, the composition may or may not include minor amounts of other elements, wherein Fe is present in amounts of at least 90%, wherein Mn is present in amounts of 0.5-6%, Si is present in amounts of 0.001-3%, C is present in amounts of 0.01 to 0.1%; and drawing the composition into a tube.

5. The method of claim 4, further comprising electropolishing the tube.

6. The method of claim 4, further comprising laser cutting the tube to include a series of elements meeting at an acute angle, the angle increasing on radial expansion of the tube.

## Patentansprüche

1. Medizinischer Stent, umfassend: einen röhrenförmigen Körper, der aus einer Metallzusammensetzung hergestellt ist, die aus Fe, Mn, Si und C besteht, wobei die Zusammensetzung geringe Mengen an anderen Elementen einschließen kann oder nicht, wobei Fe in Mengen von mindestens 90 % vorhanden ist, wobei Mn in Mengen von 0,5 bis 6 % vorhanden ist, Si in Mengen von 0,001 bis 3 % vorhanden ist, C in Mengen von 0,01 bis 0,1 % vorhanden ist.

2. Medizinischer Stent nach Anspruch 1, wobei die Mengen an Mn, Si und C in der Zusammensetzung wie folgt sind: 2 bis 3 % Mn, 0,1 bis 0,3 % Si und 0,01 bis 0,03 % C.

3. Apparat, umfassend: einen Katheter und einen Stent gemäß Anspruch 1, der auf dem Katheter angebracht ist, wobei der Katheter so vorgesehen ist, dass er den Stent durch plastische Verformung expandiert.

4. Verfahren zum Herstellen des Stents nach Anspruch 1, umfassend:
Bereitstellen einer Metallzusammensetzung, bestehend aus Fe, Mn, Si und C, wobei die Zusammensetzung geringe Mengen an anderen Elementen einschließen kann oder nicht,
wobei Fe in Mengen von mindestens 90 % vorhanden ist, wobei Mn in Mengen von 0,5 bis 6 % vorhanden ist, Si in Mengen von 0,001 bis 3 % vorhanden ist, C in Mengen von 0,01 bis 0,1 % vorhanden ist; und
Ziehen der Zusammensetzung zu einer Röhre.

5. Verfahren nach Anspruch 4, ferner umfassend Elektropolieren der Röhre.

6. Verfahren nach Anspruch 4, ferner umfassend Laserschneiden der Röhre, so dass diese eine Reihe von Elementen einschließt, die sich in einem spitzen Winkel treffen, wobei der Winkel bei radialer Expansion der Röhre zunimmt.

## Revendications

1. Stent médical, comprenant : un corps tubulaire formé d'une composition métallique constituée par Fe, Mn, Si et C, la composition pouvant inclure ou non des quantités mineures d'autres éléments, où Fe est présent à des teneurs d'au moins 90 %, où Mn est présent à des teneurs de 0,5 à 6 %, Si est présent à des teneurs de 0,001 à 3 %, C est présent à des teneurs de 0,01 à 0,1 %.

2. Stent médical selon la revendication 1, où les teneurs de Mn, Si et C dans la composition sont les suivantes : 2 à 3 % de Mn, 0,1 à 0,3 % de Si et 0,01 à 0,03 % de C.

3. Appareil comprenant : un cathéter et un stent selon la revendication 1 qui est monté sur le cathéter, le cathéter étant disposé de sorte à dilater le stent par déformation plastique.

4. Procédé de formation du stent selon la revendication 1, comprenant :
le fait de se munir d'une composition métallique constituée par Fe, Mn, Si et C, la composition pouvant inclure ou non des quantités mineures d'autres éléments, où Fe est présent à des teneurs d'au moins 90 %, où Mn est présent à des teneurs de 0,5 à 6 %, Si est présent à des teneurs de 0,001 à 3 %, C est présent à des teneurs de 0,01 à 0,1 % ; et
l'étirement de la composition de sorte à former un tube.

5. Procédé selon la revendication 4, comprenant en outre l'électropolissage du tube.

6. Procédé selon la revendication 4, comprenant en outre la découpe au laser du tube pour inclure une série d'éléments se rencontrant à un angle aigu, l'angle augmentant lors de la dilatation radiale du tube.
